# EUROPEAN PATENT APPLICATION

(11) **EP 4 763 274 A1**
(43) Date of publication of application: **24.06.2026**
(21) Application number: 24220699.3
(22) Date of filing: 17.12.2024
(51) Int. Cl.: A61N 1/375, H01R 13/52

(54) **COMBINATION OF SCREW AND SEALING PLUG FOR IS-1/DF-1, IS-4/DF-4 PLUG HOLES, WHICH ENABLES ELECTRICAL ISOLATION AND FIXES THE ELECTRODE**

(71) Applicant: BIOTRONIK SE & Co. KG, 12359 Berlin (DE)
(72) Inventor: Binias, Sofia, 10587 Berlin (DE); Fritsch, Jan, 91301 Forchheim (DE); Schmidt, Achim, 55268 Nieder-Olm (DE); Kloth, Christoph, 12529 Schönefeld (DE); Völker, Kerstin, 12621 Berlin (DE); Bohmeyer, Martin, 15366 Neuenhagen (DE); Litzke, Jan, 13507 Berlin (DE); Wilknitz, Andreas, 16359 Biesenthal (DE); Pritzel, Oliver, 12105 Berlin (DE); Große, Ines, 14612 Falkensee (DE)
(74) Representative: Biotronik Corporate Services SE

(57) **Abstract**

The present invention relates to an implantable medical device (1), comprising: a housing (2) for accommodating an electrical circuit, and a header (10) connected to the housing (2), the header (10) comprising a connector socket (11) configured to accommodate a plug (3) of an electrode lead and comprising a connector (5), wherein the header (10) further comprises a screw (4) screwed into an inner thread (30) of the connector (5) to fix the plug when the plug is inserted in the socket (11), wherein the header (10) comprises an opening (31) for accessing the inner thread (30), and wherein the header (10) comprises a sealing member (40) configured to be accommodated in the opening (31) to seal the opening (31). According to the invention, said opening (31) is an undercut-free opening for facilitating insertion of the sealing member (40) into said opening (31).

## Description

The present invention relates to an implantable medical device comprising a header.

Such headers comprise at least one connector socket with at least one connector for receiving a plug or lead connector of an electrode lead. Particularly, to ensure proper functioning, the plug has to be fixed in the connector socket. This is usually done by fixing the plug with a screw screwed into a thread arranged in the connector and being assessable from an outside via an opening of the header. The end of the screw when properly screwed into the inner thread then presses against the plug residing in the connector socket and therefore secures the plug in the connector socket. The opening for accessing the screw and inner thread then has to be sealed by a sealing plug.

Known solutions require to mount the sealing plug as a component in said opening so that it engages behind an undercut of the opening of the header. The threaded screw is mounted separately.

However, the production of the sealing plug and the undercut in the header are complex. The process of fitting the sealing plug in production is often time-consuming. Primarily, the undercut serves for holding the sealing plug in position and to minimize the risk of an unwanted release of the sealing plug from the opening.

Based on the above, the problem to be solved by the present invention is to provide a simple and cost-effective process allowing to securely fix the plug in the connector socket while mitigating at the same time the risk of a release of the sealing plug from said opening.

This problem is solved by an implantable medical device having the features of claim 1. Preferred embodiments of this aspect of the present invention are stated in the corresponding dependent claims and are described below.

According to claim 1, and implantable medical device is disclosed, comprising:
- a housing for accommodating an electrical circuitry, and
- a header connected to the housing, the header comprising a connector socket configured to receive a plug or lead connector of an electrode lead and comprising a connector, wherein the header further comprises a screw screwed into an inner thread of the connector to fasten the plug when the plug is inserted in the connector socket, wherein the header comprises an opening for accessing the inner thread, and wherein the header comprises a sealing member configured to be accommodated in the opening to seal the opening.

According to the present invention, said opening is an undercut-free opening for facilitating insertion (and particularly disassembly) of the sealing member into said opening.

Particularly, according to one embodiment, the implantable medical device may be an implantable cardiac pacemaker or an implantable cardioverter defibrillator.

Particularly, the connector socket, particularly the connector is configured to receive and to electrical contact element a plug of an electrode lead, wherein the connector arranged adjacent the opening. Furthermore, the connector socket, particularly the connector is particularly configured to provide electrically conductive connections to the electronic circuitry accommodated in the housing, wherein the header is arranged on the housing, wherein the electrically conductive connection between the connector socket and the electronic circuitry may be established through an electrical feedthrough that hermetically seals the housing and comprises feedthrough pins embedded in or extending through an electrically insulating body, e.g., made from a ceramic such as alumina (Al₂O₃), a glass, a glass solder or a plastic material. At least one feedthrough pin, e.g., made from platinum, iridium, niobium, platinum/iridium, or titanium, may be connected to a connector (e.g. socket) integrated into the header body. In this way, e.g. a plug of an electrode lead inserted into the connector may be connected to the electronic circuitry. The header may be arranged on the housing so as to cover the electrical feedthrough. Particularly, the at least one feedthrough pin is hermetically joined with the electrically insulating body, particularly by brazing. Particularly, the electrically insulating body is hermetically joined with the housing, particularly by brazing.

Particularly, in one embodiment, the opening extends from an outer surface of the header in an axial direction to the inner thread, wherein the opening preferably comprises a constant inner diameter throughout its extension from the outer surface to the inner thread.

Furthermore, according to one embodiment, a header body of the header, the header body comprising said opening and accommodates said connector socket, preferably is an injection molded header body, and/or is formed out of or comprises a thermoplastic polyurethane (TPU).

Furthermore, according to one embodiment, the sealing member is an elastically deformable sealing plug.

In a further embodiment, the sealing member comprises a lateral circumferential outer surface having an outer diameter being larger than an inner diameter of the opening, so that the sealing member comprises a press-fit with the opening when inserted into the opening. Particularly, the lateral circumferential outer surface is cylindrical.

According to one embodiment, the lateral circumferential outer surface is tapered, particularly a lower portion thereof, to facilitate insertion of the sealing member into the opening, wherein the sealing member comprises an upper portion configured to tightly butt against an inside of the opening when the sealing member is inserted into the opening.

Furthermore, according to one embodiment of the present invention, the sealing member comprises two or three circumferential sealing lips which are configured to tightly butt against the inside of the opening of the header when the sealing member is inserted into the opening.

Further, in one embodiment, the sealing member comprises a self-sealable through-opening allowing insertion of a tool through the through-opening of the sealing member to facilitate screwing of the screw into the inner thread with a tool (e.g. with an Allen key).

According to one embodiment, the sealing member is a separate element with respect to the screw (particularly not directly connected to the screw), wherein particularly the sealing member comprises an aperture for accommodating a head of the screw when the sealing member is inserted into the opening of the header.

According to one alternative embodiment of the present invention, the sealing member is connected to the screw.

Furthermore, according to one embodiment, the sealing member comprises or is formed out of one of the following materials: silicone, thermoplastic polyurethane.

Furthermore, in one embodiment, the sealing member comprises an undercut behind which a head of the screw engages to establish a connection with the sealing member.

In an alternative embodiment, the sealing member is permanently bonded to the screw.

According to one embodiment, the screw comprises a circumferential groove between a head of the screw and an outer thread of the screw, the outer thread being configured to be screwed into the inner thread of the connector.

Furthermore, according to one embodiment of the present invention, the screw comprises or is formed out of one of the following materials: a metal, particularly stainless steel, e.g., MP35N, or titanium, or a plastic material, particularly PEEK (polyether ether ketone), TPU (thermoplastic polyurethane), PE (polyethylene), PMMA (polymethylmethacrylate) or PSU (polysulfone).

Further, in one embodiment, the screw comprises a head and a shaft, the shaft comprising on outer thread configured to be screwed into the inner thread of the connector, and wherein the head is connected to the shaft, wherein the sealing member is a sealing ring surrounding the shaft and being arranged adjacent to the head. Particularly, the sealing ring is formed out of one of the following materials: silicone, PU (polyurethane).

Furthermore, according to one embodiment, the head is bonded to the shaft, particularly molded onto the shaft (particularly by way of injection molding), wherein particularly the shaft is formed out of a metal and the head is formed out of a plastic material. In embodiment, the metal is one of: steel, particularly stainless steel, e.g., MP35N, or titanium. In one embodiment, the plastic material of the head is one of: PEEK, TPU, PE, PMMA, PSU, Silicon.

According to one embodiment, the head is integrally connected to the screw (particularly by way of injection molding), wherein particularly the head and the shaft are formed out of a plastic material. In one embodiment, said plastic material of the head is one of: PEEK, TPU, PE, PMMA, PSU, silicone. In one embodiment, said plastic material of the shaft is one of: PEEK, TPU, PE, PMMA, PSU. Particularly, the shaft can be formed out of the same plastic material as the head.

Furthermore, in a further embodiment, the head is a rigid body and/or the head comprises an aperture (particularly in form of a blind hole) for engaging with a tool (such as an Allen key), e.g., in a form fitting manner, for allowing to screw the outer thread of the shaft into said inner thread of the header.

In the following, embodiments of the aspects of the present invention as well as further features and advantages of the present invention shall be described with reference to the Figures, wherein
- Fig. 1: shows an embodiment of an implantable medical device according to the present invention,
- Fig. 2: shows a cross-sectional view of a portion of a header of the implantable medical device along the line II-II of Fig. 1,
- Fig. 3A: shows a lateral view of a sealing member and a screw of an embodiment of the implantable medical device, wherein the sealing member engages with the screw,
- Fig. 3B: shows a cross-sectional view of the sealing member and screw shown in Fig. 3A,
- Fig. 3C: shows a top view of the sealing member shown in Fig. 3A and 3B,
- Fig. 4A: shows a lateral view of a sealing member and a screw of a further embodiment of the implantable medical device, wherein sealing member and screw are bonded to one another,
- Fig. 4B: shows a cross-sectional view of the sealing member and screw shown in Fig. 4A,
- Fig. 4C: shows a top view of the sealing member shown in Fig. 4A and 4B,
- Fig. 5A: shows a lateral view of a screw of a further embodiment of the implantable medical device, the screw comprises a head bonded to a shaft of the screw and an annular sealing member encompassing the shaft,
- Fig. 5B: shows a cross-sectional view of the screw and its sealing member shown in Fig. 5A,
- Fig. 5C: shows a top view of the head of the screw shown in Fig. 5A and 5B,
- Fig. 6A: shows a lateral view of a sealing member of a further embodiment of the implantable medical device, the sealing member comprising two sealing lips; particularly, this sealing member may also be used in the embodiments of Figs. 3A-C and 4A-C,
- Fig. 6B: shows a cross-sectional view of the sealing member shown in Fig. 6A,
- Fig. 6C: shows a top view of the sealing member shown in Fig. 6A and 6B,
- Fig. 7A: shows a lateral view of a sealing member and a screw of a further embodiment of the implantable medical device, wherein the sealing member corresponds to the one shown in Figs. 6A-C and the screw may correspond to the one shown in Figs. 3A-C,
- Fig. 7B: shows a cross-sectional view of the sealing member and screw shown in Fig. 7A,
- Fig. 7C: shows a top view of the sealing member shown in Fig. 7A and 7B,
- Fig. 8A: shows a lateral view of a sealing member of a further embodiment of the implantable medical device, the sealing member comprising three sealing lips; particularly, this sealing member may also be used in the embodiments of Figs. 3A-C and 4A-C, and
- Fig. 8B: shows a cross-sectional view of the sealing member shown in Fig. 8A.

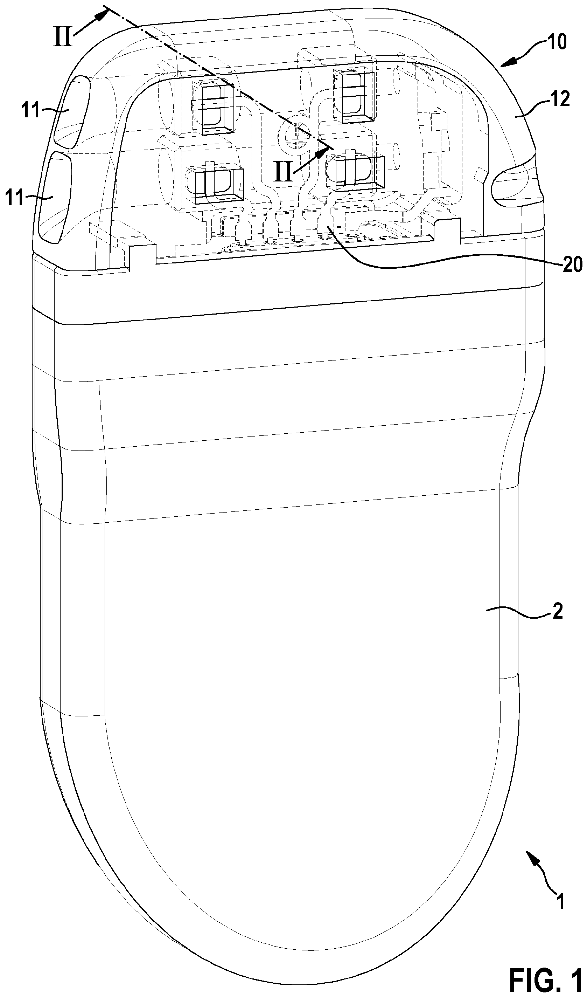

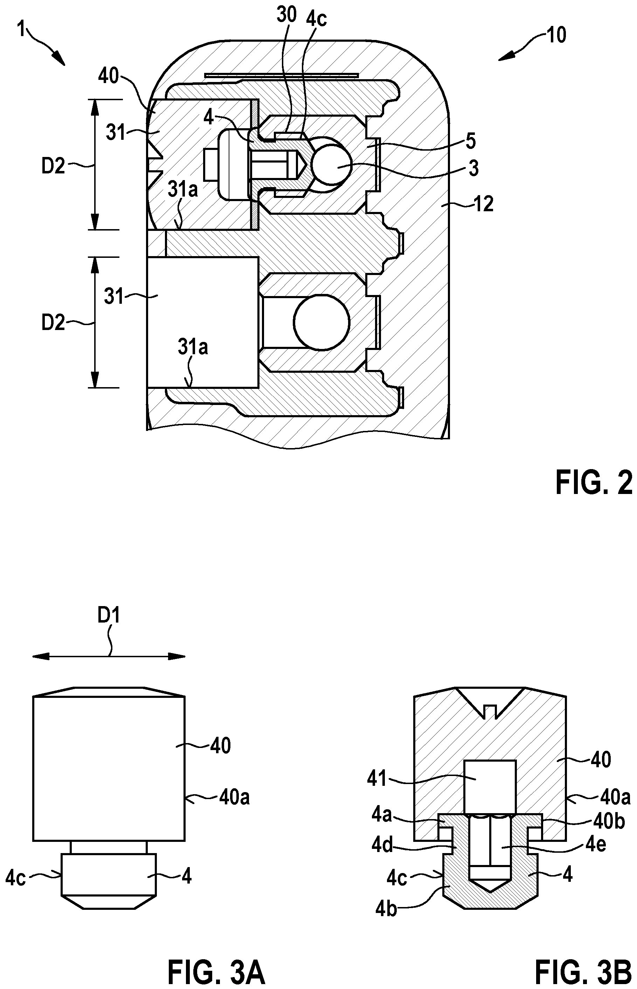

Fig. 1 shows in conjunction with Fig. 2 an embodiment of an implantable medical device 1 according to the present invention. According thereto, the implantable medical device 1 comprises a housing 2 configured for accommodating an electrical circuitry of the device 1. The device 1 further comprises a header 10 that may arranged adjacent to an electrical feedthrough 20 of the device 1 or may cover the electrical feedthrough 20, wherein the feedthrough 20 provides an electrical connection to the circuitry from outside the housing 2. Particularly, the electrical feedthrough comprises an electrically insulating body, e.g., made form a ceramic such as alumina, and one or more feedthrough pin extending through the insulating body. Particularly, the header 10 may comprises a header body 12 mounted to the housing 2, wherein the header body 12 accommodates at least one connector socket 11 with a least one connector 5 (here e.g. two connectors 5). Particularly, the respective connector socket 11 may be configured to receive one of the following plugs or comply to the following standards: IS-1/DF-1, IS-4/DF-4.

Particularly, the implant 1 can be an implantable medical device that provides therapy in form of electrical stimulation. In this case, the socket(s) 11 can be configured to receive a plug of an electrode lead via which therapy can be applied to a patient and/or sensing can be achieved. The connector sockets 11 may be electrically connected to the electrical circuit accommodated in the housing 2 via the electrical feedthrough 20. Particularly, the header body 12 may be an injection molded header body 12, wherein particularly the header body 12 can be injection molded out of a thermoplastic polyurethane (TPU).

As may be inferred from the cross-section shown in Fig. 2, a connector socket 11 may comprise connector 5 having an inner thread 30. Furthermore, the header 10 comprises at least one opening 31 through which an inner thread 30 the is accessible, wherein the inner thread 30 receives an outer thread 4c of a screw 4 by means of which a plug 3 inserted into an associated socket 11 (cf. Fig. 1), and particularly received in the connector 5, may be fastened, e.g. by way of clamping the plug down with the screw 4. Particularly, the at least one opening 31 extends from an outer surface 10a of the header 10 in an axial direction x to the inner thread 30. According to the present invention, the at least one opening 31 is an undercut-free opening having a constant inner diameter D in particular, which allows an uncomplicated insertion of the sealing member 40 into the at least one opening 31.

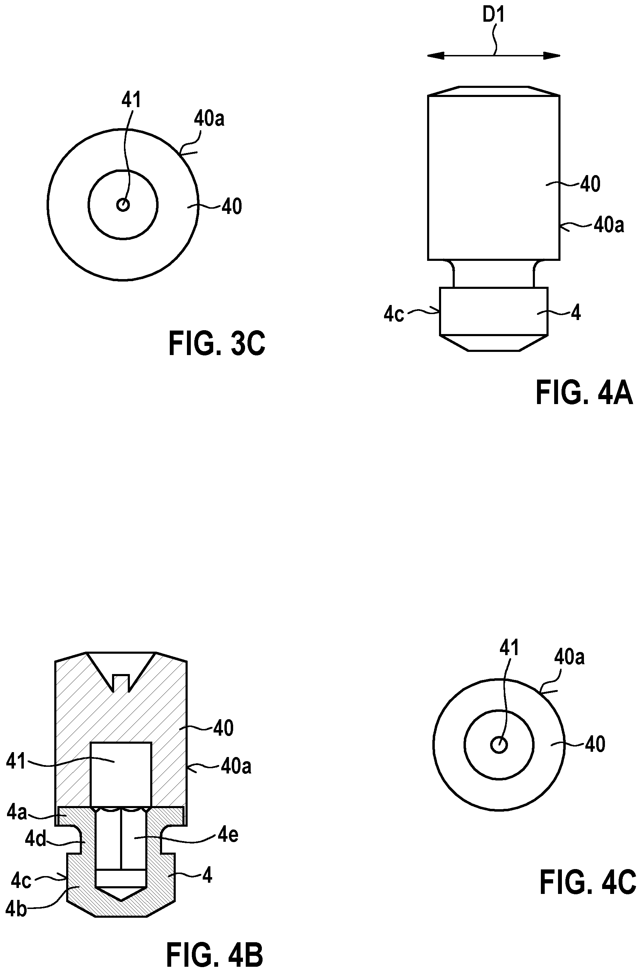

Figs. 3A to 8B now show different embodiments of the present invention relating to the sealing member 40 and the screw 4 that may be used to provide fixation of a plug 3 when residing in a connector socket 11, more particularly in a connector 5 of the connector socket, as well as proper sealing of the opening 31 through which the screw 4 is mounted into the corresponding inner thread 30.

According to the embodiment shown in Fig. 3A-C, the sealing member 40 is an elastically deformable sealing plug 40 which may be formed out of silicone, or thermoplastic polyurethane for example. The sealing member 40 comprises a lateral circumferential outer surface 40a having an outer diameter D1 being larger than an inner diameter D2 of the opening(s) 31 of the header 10 / header body 12 shown in Fig. 2, so that the sealing member 40 comprises a press-fit with the opening 31 when inserted into the opening 31, or in other words, sealing member 40 is retained in the opening 31 by a press-fit when being inserted in the opening 31. Particularly, the lateral circumferential outer surface 40a is cylindrical. Alternatively, the lateral circumferential outer surface may be tapered (e.g. to form a truncated cone) to facilitate insertion of the sealing member 40 into the opening. Here, the sealing member 40 may be inserted into the opening 31 with the thinner end ahead, while a broader upper portion tightly butts against an inside 31a of the opening 31 when the sealing member 40 is fully inserted into the opening 31.

Furthermore, as shown in Figs. 3A-B, the sealing member 40 may be connected to the screw 4. The screw 4 may comprise a head 4a integrally connected to a shaft 4b of the screw 4 on which an outer thread 4c is formed that is configured to be screwed into the inner thread 30 of the connector5. For connecting the sealing member 40 to the screw 4, the sealing member 40 may comprise an undercut 40b behind which said head 4a of the screw 4 extends (e.g. in a form-fitting manner) to establish a mechanical connection with the sealing member 40. Furthermore, the screw 4 may comprise a circumferential groove 4d formed in the shaft 4b adjacent the head 4a which may be utilized to achieve a more pronounced engagement of the head 4a in the sealing member 40.

Furthermore, the sealing member 40 may comprises a self-sealable through-opening 41 allowing insertion of a tool through the through-opening 41 of the sealing member 40 to facilitate screwing of the screw 4 into the inner thread 30 with an appropriate tool that may operatively interact with the head 4a of the screw 4. Particularly, the screw 4 may comprise a recess 4e for receiving the tool.

The embodiment shown in Figs. 3A-C allows to seal the opening 31 even if the sealing member 31 comes loose from the screw 4. Further, no undercut is necessary in the opening 31, instead easy assembly is facilitated by means of an isodiametric opening 31. Furthermore, this allows to assemble and disassemble the sealing member / plug 40 without damaging the sealing member 40 by an undercut in the header (material shearing with normal sealing plugs due to material protrusion).

Fig. 4A-C shows a further embodiment according to the present invention. Also here, the sealing member 40 is an elastically deformable sealing plug 40 which may be formed out silicone for example. The sealing member 40 comprises a lateral circumferential outer surface 40a having an outer diameter D 1 that may be larger than an inner diameter D2 of the opening(s) 31 of the header 10 / header body 12 shown in Fig. 2, so that the sealing member 40 may seal the opening 31 when inserted into the opening 31. Particularly, the lateral circumferential outer surface 40a is cylindrical.

Furthermore, in contrast to Figs. 3A-B, the sealing member 40 is permanently bonded to the screw 4, particularly to a head 4a of the screw 4. This bond may be established by molding the sealing member 40 onto the head 4a or by using other adhesives.

Again, the sealing member 40 may comprises a self-sealable through-opening 41 allowing insertion of a tool through the through-opening 41 of the sealing member 40 to facilitate screwing of the screw 4 into the inner thread 30 with an appropriate tool that may operatively interact with the head 4a of the screw 4, particularly with a recess 4e formed therein. Furthermore, the screw 4 may comprise a circumferential groove 4d formed in the shaft 4b adjacent the head 4a.

Particularly, the embodiment shown in Fig. 4A-C does also not require an opening 31 with an undercut which facilitates easy assembly due to the isodiametric geometry of the opening 31. Furthermore, assembly and disassembly are possible without damaging the sealing member / plug 40 by an undercut in the header (material shearing with normal sealing plugs due to material protrusion).

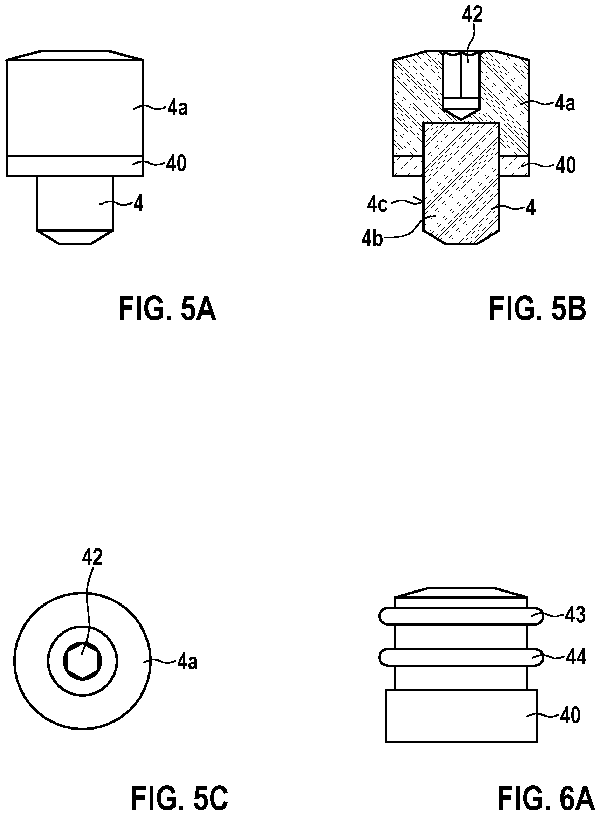

Fig. 5A-C shows a further embodiment according to the present invention, wherein the screw 4 comprises a head 4a and a shaft 4b, the shaft 4b comprising an outer thread 4c configured to be screwed into the inner thread 30 of the connector 5, and wherein the head 4a is connected to the shaft 4b. Furthermore, here, the sealing member 40 is a sealing ring surrounding the shaft 4b and being arranged adjacent to the head 4a.

According to a first variant of this embodiment, the head 4a and the shaft 4b are integrally connected to one another, i.e., form a monolithic body. Particularly, the head 4a and the shaft 4b may both be formed out of PEEK for example. The annular sealing member 40 can be formed out of silicone.

Thus, in contrast to the embodiments described above, the head 4a does not form a sealing plug, but is designed to be inserted into the opening 31 in a form-fitting fashion (or with a little play) wherein a sealing effect is now established by the sealing ring 40 that is pressed predominantly against the bottom of opening 31 and thus seals the inner thread 30.

Furthermore, the head 4a may be a rigid body and may comprise an aperture 42, e.g. in form of a blind hole, configured to engage with a suitable tool (e.g. in a form fitting manner) for allowing to screw the outer thread 4c of the shaft 4b of the screw 4 into said inner thread 30 of the header 10.

In an alternative second variant of the screw 4 shown in Figs. 5A-C, the head 4a is bonded to the shaft 4b, e.g., by way of injection molding. Here, the shaft 4b may be formed out of a metal, and the head 4a is formed out of a plastic material, such as PEEK. The remaining features of the first variant may apply to the second variant as well.

Advantageously, the embodiment according to Figs. 5A-C allows to establish a seal via a sealing ring 40 and a (plastic or metal) screw 4, so that the flexible sealing plug may be omitted. This allows an easier assembly in the opening 31 (isodiametric) without damaging a portion of the screw 4 as there are no undercuts in the opening 31.

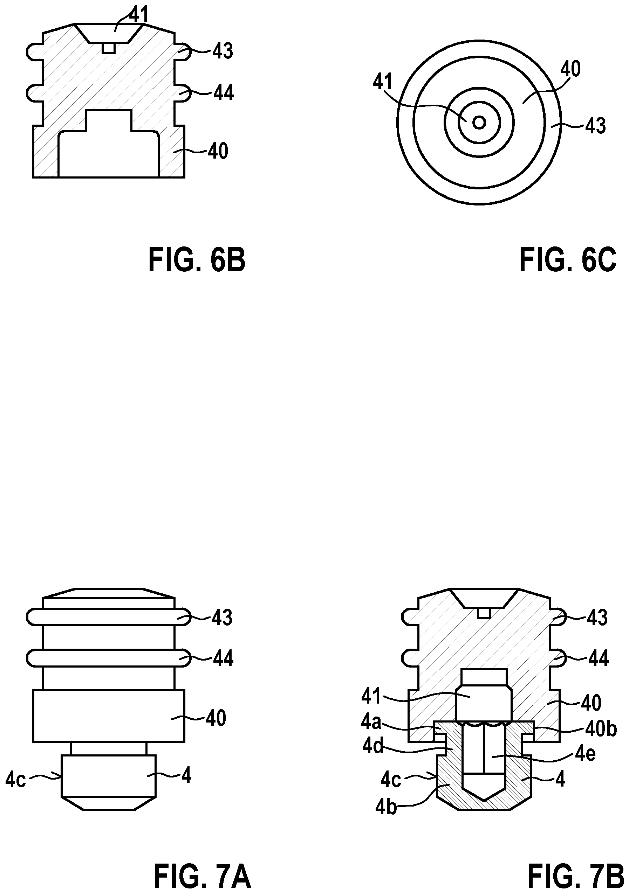

Figs. 6A-C show a further embodiment of the present invention, wherein here the sealing member 40 forms a separate sealing plug comprising two circumferential sealing lips 43, 44 that contact the inside 31a of the opening 31 when the sealing member 40 is inserted into the opening 41. Particularly, the sealing member / plug 40 can comprise an aperture 46, for accommodating a head 4a of a separate screw 4 that is used to fix the plug 3 in a socket 11 of the header 10.

Advantageously, the sealing member 40 properly seals the opening 31 even in case the screw 4 is loose and does not fix the plug. As there is no undercut required/present in the opening 31 of the header 10 an easy insertion of the sealing member 40 into the opening 31 (isodiametric) is possible. Furthermore, assembly and disassembly of the sealing member 40 are possible without damaging the sealing member 40 by an undercut in the opening 31 of header 10 (material shearing with normal sealing plugs due to material protrusion).

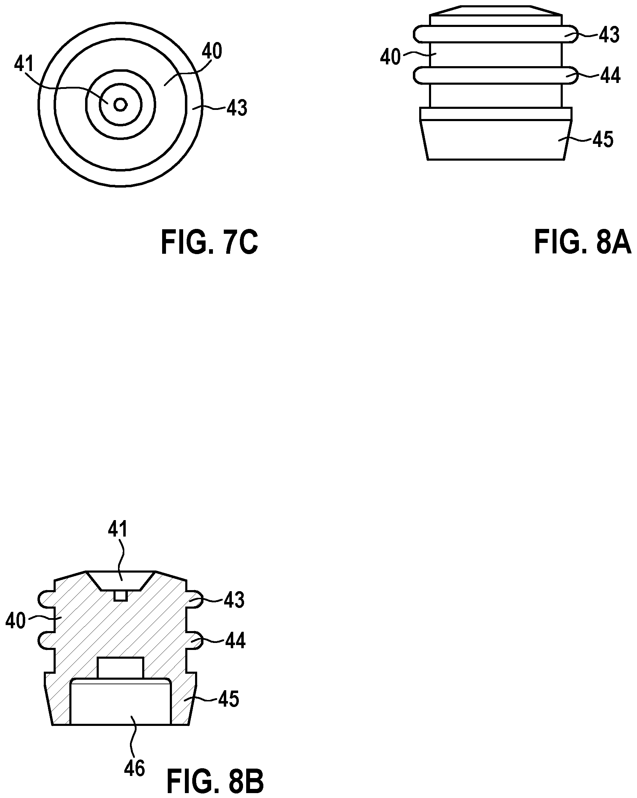

However, the sealing member 40 shown in Figs. 6A-C can also be used as the sealing member 40 of the embodiments shown in Figs. 3A-C and Figs. 4A-C. Particularly, the embodiment shown in Figs. 7A-C shows the application of the sealing member 40 of Figs. 6A-C to the screw 4 of Figs. 3A-C.

Finally, Fig. 8A shows a modification of the embodiment shown in Fig. 6A-C, wherein here the sealing member / plug 40 comprises three circumferential sealing lips 43, 44, 45, wherein the third sealing lip 45 arranged at an end of the sealing member 40 may be tapered to ease insertion of the sealing member 40 into the opening 31. Further, the third sealing lip 45 may comprise a larger width in the axial (insertion) direction of the sealing member 40 than the first and the second lip 43, 44.

Advantageously, the sealing member 40 also properly seals the opening 31 even in case the screw 4 is loose and does not fix the plug. As there is no undercut required/present in the opening 31 of the header 10 an easy insertion of the sealing member 40 into the opening 31 (isodiametric) is possible. Furthermore, particularly, a tapered shape of the third sealing lip 45 improves assembly of the sealing member 40. Both assembly and disassembly of the sealing member 40 are possible without damaging the sealing member 40 by an undercut in the opening 31 of header 10 (material shearing with normal sealing plugs due to material protrusion).

Again, the sealing member 40 shown in Fig. 8A-B may also be used as the sealing member 40 of the embodiments shown in Figs. 3A-C and Figs. 4A-C.

## Claims

1. An implantable medical device (1), comprising:
- a housing (2) for accommodating an electrical circuitry,
- a header (10) connected to the housing (2), the header (10) comprising a connector socket (11) configured to accommodate a plug (3) of an electrode lead and comprising a connector (5), wherein the header (10) further comprises a screw (4) screwed into an inner thread (30) of the connector (5) to fasten the plug (3) when the plug (3) is inserted in the connector (5), wherein the header (10) comprises an opening (31) for accessing the inner thread (30), and wherein the header (10) comprises a sealing member (40) configured to be accommodated in the opening (31) to seal the opening (31),
**characterized in that**
said opening (31) is an undercut-free opening for facilitating insertion of the sealing member (40) into said opening (31).

2. The implantable medical device according to claim 1, wherein the sealing member (40) is an elastically deformable sealing plug.

3. The implantable medical device according to claim 1 or 2, wherein the sealing member (40) comprises a lateral circumferential outer surface (40a) having an outer diameter (D1) being larger than an inner diameter (D2) of the opening (31) in a unconstrained condition, so that the sealing member (40) is retained in the opening (31) by a press-fit when inserted into the opening (31).

4. The implantable medical device according to claim 3, wherein the lateral circumferential outer surface (40a) is cylindrical.

5. The implantable medical device according to claim 3, wherein the lateral circumferential outer surface is tapered, particularly at least a lower portion of the lateral surface, to facilitate insertion of the sealing member (40) into the opening (31), wherein the sealing member (40) comprises an upper portion configured to tightly butt against an inside (31a) of the opening (31) when the sealing member (40) is inserted into the opening (31).

6. The implantable medical device according to one of the claims 1 to 3, wherein the sealing member (40) comprises two or three circumferential sealing lips (43, 44, 45).

7. The implantable medical device according to one of the preceding claims, wherein the sealing member (40) comprises a self-sealable through-opening (41) allowing insertion of a tool through the through-opening (41) of the sealing member (40) to facilitate screwing of the screw (4) into the inner thread (30) with a tool.

8. The implantable medical device according to one of the preceding claims, wherein the sealing member (40) is a separate element with respect to the screw (4), wherein particularly the sealing member (40) comprises an aperture (46) for accommodating a head of the screw when the sealing member (40) is inserted into the opening (31) of the header (10).

9. The implantable medical device according to one of the claims 1 to 7 sealing member (40) is connected to the screw (4).

10. The implantable medical device according to one of the preceding claims, wherein the sealing member (40) comprises an undercut (40b) behind which a head (4a) of the screw (4) engages to establish a connection with the sealing member (40).

11. The implantable medical device according to one of the claims 1 to 9, wherein the sealing member (40) is bonded to the screw (4).

12. The implantable medical device according to claim 1, wherein the screw (4) comprises a head (4a) and a shaft (4b), the shaft (4b) comprising an outer thread (4c) configured to be screwed into the inner thread (30) of the connector (5), and wherein the head (4a) is connected to the shaft (4b), wherein the sealing member (40) is a sealing ring surrounding the shaft (4b) and being arranged adjacent to the head (4a).

13. The implantable medical device according to claim 12, wherein the head (4a) is bonded to the shaft, wherein particularly the shaft (4b) is formed out of a metal and the head (4a) is formed out of a plastic material.

14. The implantable medical device according to claim 12, wherein the head (4a) is integrally connected to the screw (4), wherein particularly the head (4a) and the shaft (4b) are formed out of a plastic material.

15. The implantable medical device according to one of the claims 12 to 14, wherein the head (4a) is a rigid body and/or wherein the head (4a) comprises an aperture (42) for engaging with a tool for allowing to screw the outer thread (4c) of the shaft (4b) into said inner thread (30) of the header (10).
